# EUROPEAN PATENT SPECIFICATION

(11) **EP 2 169 048 B1**
(45) Date of publication and mention of the grant of the patent: **21.03.2018**
(21) Application number: 09010406.8
(22) Date of filing: 12.08.2009
(51) Int. Cl.: C12M 1/36

(54) **System and method for cultivating cells**
System und Verfahren zur Kultivierung von Zellen
Système et procédé de culture de cellules

(30) Priority: 29.09.2008 JP 2008251406
(43) Date of publication of application: 31.03.2010
(73) Proprietor: Hitachi, Ltd., Chiyoda-ku Tokyo 100-8280 (JP)
(72) Inventor: Shibuya, Keisuke, Tokyo 100-8220 (JP); Haga, Ryoichi, Tokyo 100-8220 (JP); Namba, Masaru, Tokyo 100-8220 (JP)
(74) Representative: Strehl Schübel-Hopf & Partner

(56) References cited:
- EP-A1- 1 816 188
- JP-A- 2008 178 344
- US-A- 4 064 015
- US-A- 4 629 686

## Description

### BACKGROUND OF THE INVENTION

### Field of the Invention

The present invention relates to a system for cultivating cells, for example, which produce a substance to be a principle component of pharmaceuticals, etc.

### Background Art

Some pharmaceuticals, including antibiotic drugs, contain a cell-produced substance as a principle component. Since such a substance is produced by, for example, the secretion from animal cells, the substance can be obtained by cultivating animal cells, and isolating and purifying the intended substance secreted in the culture solution. Cells grow by repeated divisions at the step of cultivating cells, but cell death is caused in increasing number of the cells when culture environment deteriorates. When the culture environment continues to deteriorate, all the cells die in the end. Examples of the factors affecting culture environment include mechanical cell breakage by stirring, nutrients depletion, accumulation of ammonia and lactic acid secreted by the cells, etc.

Cell culture methods are classified into batch culture, continuous culture (perfusion culture), and fed-batch culture (semibatch culture). Batch culture is a method of feeding fresh medium each time, and a strain is inoculated thereon with the medium being fixed until the harvest. The quality of individual culture is unstable, but the risk of contamination is dispersed and reduced. Continuous culture is a method of feeding medium to a culture system at a constant rate and simultaneously draining an equivalent amount of the culture solution. This method is characterized in that constant culture environment is always easily maintained, whereby the productivity is stable. On the other hand, it poses a drawback in persisting contamination once it is caused. Fed-batch culture is a method of feeding medium itself or a specific component in the medium during culture and leaving the resulting products unharvested until the culture is completed. Fed-batch culture is carried out with purposes of optimizing the reproductivity by regulating a cell density and maintaining the productivity by diluting noxious substances accumulated in the culture solution.
In fed-batch culture, a high cell concentration can be achieved due to the development of fed-batch strategy in which a fed-batch method of feed medium is adapted so as not to deplete nutrients and the accumulation of noxious metabolites is prevented for achieving a high density effectively using energy sources by utilizing a carbon source (glucose), one of the energy sources, and mainly shifting the lactic acid metabolism to complete oxidization by TCA cycle. For example, JP Patent Publication (Kokai) No. 2007-244341 A discloses a method for cultivating cells in which the consumptions of glucose and glutamine in the culture solution after a determined time lapse are estimated and a glutamine concentration is maintained within a given range based on the estimations. Further, JP Patent Publication (Kohyo) No. 62-503146 A (1987) discloses a method for cultivating cells wherein a feed medium containing a carbon source and amino acid is continuously fed until the death phase of the culture.

A system for cultivating cells according to the preamble of claim 1 is disclosed in the documents US 4 064 015 A, JP 2008 178344 A and US 4 629 686 A.

### SUMMARY OF THE INVENTION

Even any of the culture methods described above posed a problem of impaired production efficiency due to cell death such as apoptosis, etc., caused by nutrients depletion and the increased accumulation of ammonia and lactic acid caused by inefficient metabolism in an excessive nutrient concentration. To prevent the inefficient metabolism in an excessive nutrient concentration, a fed-batch culture has been developed wherein a nutrient concentration is maintained at a bare minimum concentration and the control is performed using a feed medium having a single composition ratio determined by the average of the intracellular metabolic changes throughout the entire culture processes. However, such a method caused malnutrition because the cell state varies during the culture processes and each nutrient consumption rate required for the growth accordingly changes, thereby varying the optimized values of the nutrient concentration.

In view of the above circumstances, the object of the present invention is to provide a system and a method for cultivating cells by which a state of a cell during culture can be followed and an intended product can be produced with a high yield by adding a feed medium suitable for the state of the cell.

This object is achieved by the system and the method according to claims 1 and 2.

The system for cultivating cells of the present invention which has accomplished the object described above is provided with a bioreactor/fermentor for cultivating cells to be cultivated, a measuring device for measuring the culture cells being cultivated in the bioreactor/fermentor or a component contained in the culture solution, and a control device for selecting a feed medium to be added to the bioreactor/fermentor from two or more feed media having different composition ratios based on the culture cells state determined by a measured value obtained by the measuring device.

According to the system for cultivating cells of the present invention, a feed medium can be selectively added in accordance with the state of a cell being cultivated. More specifically, in the system for cultivating cells of the present invention, the control device described above enables the inhibition of nutritional imbalance by designing two or more feed media depending on the state of culture cells.

In the system for cultivating cells of the present invention, the control device analyzes a metabolic change of culture cells based on the component contained in the culture solution measured by the measuring device as the culture cells state. The control device analyzes a metabolic change of culture cells using an intracellular metabolic flux analysis. As the above intracellular metabolic flux analysis, it is preferable to employ an analysis method using Elementary Metabolite Unit (EMU). The control device can analyze a specific growth rate of culture cells based on a viable cell count to determine as a state of the culture cells. For example, the above measuring device preferably measures a glucose concentration and a lactose concentration in the culture solution and the control device preferably estimates a viable cell count based on changes in the glucose concentration and the lactose concentration as indicators. The control device analyzes apoptosis of culture cells based on the component contained in the culture solution measured by the measuring device to determine as a state of the culture cells. The above control device preferably selects a feed medium to enhance the nutrient concentration of the culture solution when a dead cell proportion is 10% or more based on the result of an apoptosis analysis. For example, a caspase digest and/or caspase, as a component contained in the culture solution, is measured, and the apoptosis of culture cells can be analyzed to determine as a state of the culture cells. The above measuring device preferably detects the caspase digest by an enzyme-linked immunosorbent assay. Examples of the above two or more feed media include those having different composition ratios of a carbon source and a nitrogen source. An example of the carbon source herein includes glucose, and an example of the nitrogen source includes glutamic acid. The composition of the above two or more feed media can be designed by a metabolic flux distribution. Further, the composition of the above two or more feed media can be designed based on an intracellular metabolic flux analysis. Examples of the component to be measured by the above measuring device include at least one selected from the group consisting of glucose, glutamic acid, lactic acid and ammonia. The above measuring device herein is preferably monitored online. The monitoring preferably analyzes the culture solution from which cells have been removed as a subject of the component analysis. More specifically, to isolate the cells contained in the culture solution, it is preferable to use a filter. For the filter, a rotating filer is preferably used. Backwashable filters are particularly preferable as the filter.

Yet furthermore, the above measuring device may be a microreaction field. More specifically, the microreaction field can, for example, quantitatively measure glucose and lactic acid in the medium, and can also quantitatively measure the above caspase digest and/or caspase. Usable examples of the microreaction field include those in which a primary antibody (anti-cytokeratin 18 antibody; M5 antibody), capable of specifically bonding to the caspase digest and/or caspase, is immobilized within a microchannel. According to the thus composed microreaction field, the caspase digest resulted from apoptosis can be quantitatively measured using biotin with the M30 antibody bonded thereto, streptavidin with horseradish peroxidase bonded thereto and TMB (3,3',5,5'-tetramethyl-benzidene), the chromogenic substrate of horseradish peroxidase. At the time of the measurement, an acid solution is mixed after the reaction of horseradish peroxidase and TMB, and an absorbance is subsequently measured. More specifically, the microreaction field is configurated so that an acid solution channel for mixing the acid solution is connected to the above-mentioned microchannel with the primary antibody immobilized thereon. For mixing the acid solution and a reaction substrate, it is preferable to allow sulfuric acid to flow so as for the mixing of the acid solution and the reaction substrate to meet T<τ (τ: the time required for pH to be lowered by the diffusion of the stop solution). It is also preferable to provide a micro valve between the microchannel for the reaction and the acid solution channel. It is further preferable to provide a gas supply channel between the microchannel for the reaction and the acid solution channel. It is furthermore preferable to provide a difference in height between the microchannel for the reaction and the acid solution channel. By structuring the microreaction field in the manner described above, the acid solution can be prevented from flowing into (including the inflow by the diffusion) the microchannel for the reaction. An intended product with high quality can be produced in good yield by maintaining the optimal environment for a state of a cell.

### BRIEF DESCRIPTION OF THE DRAWINGS

FIG. 1 is a block diagram schematically depicting the system for cultivating cells of the present invention.
FIG. 2 is a graph showing the relationship between the number of days in culture and consumption rates of glucose and glutamine.
FIG. 3 is a graph showing the relationship between specific growth rate and consumption rates of glucose and glutamine.
FIG. 4 is a conceptual drawing showing ammonia inhibition control.
FIG. 5 is a graph showing the relationship between the total consumption of glucose and lactic acid and the time integral value of viable cell count.
FIG. 6 is a graph comparing viable cell count estimated from the measurements of glucose and lactic acid with viable cell count measured by the trypan blue staining.
FIG. 7 is a conceptual drawing showing apoptosis detection method using M30 antibody.
FIG. 8 is a drawing showing the relationship between cell count in fed-batch culture and apoptosis amount.
FIG. 9 is a block diagram schematically showing an analyzer using a microreaction field.
FIG. 10 is a block diagram schematically showing an embodiment of a microreaction field.
FIG. 11 is a block diagram schematically showing another embodiment of a microreaction field.
FIG. 12 is a block diagram schematically showing yet another embodiment of a microreaction field.
FIG. 13 is a block diagram schematically showing still another embodiment of a microreaction field.
FIG. 14 is a graph showing glucose calibration curve by an analyzer using a microreaction field.
FIG. 15 is a graph showing lactic acid calibration curve by an analyzer using a microreaction field.
FIG. 16 is a graph showing apoptosis calibration curve by an analyzer using a microreaction field.
FIG. 17 is a flow diagram showing an embodiment of an aseptic sampling device.
FIG. 18 is a flow diagram showing an embodiment of the system for cultivating cells provided with the aseptic sampling device of FIG. 17.

### DESCRIPTION OF SYMBOLS

1 ... bioreactor/fermentor, 2 ... feed-medium vessel, 3 ... aseptic sampling device, 4 ... analyzer, 5 ... analysis device, 6 ... control device, 7 ... valve, 8 ... pump, 101 ... bioreactor/fermentor, 102 ... filter, 103 ... pump, 104 ... syringe, 105 ... microchannel, 106 ... chip, 107 ... absorption spectrometer, 108 ... detector, 109 ... cell, 110 ... wavelength filter, 111 ... light source, 112 ... waste fluid tank, 113 ... apoptosis detector, 114 ... recorder, 115 ... analysis device, 116 ... control device, 201 ... rotating filter, 202 ... filter, 203 ... cylinder, 204 ... filter container, 205 ... filter drive motor, 206 ... pressure gauge, 207 ... pressure gauge, 208 ... backwash water reservoir, 209 ... pressure gauge, 210 ... pressure control valve, 211 ... cylindrical gap, 212 ... filtrate channel, 213 ... rotation axis, 214 ... axial channel, 215; axial seal, 216a ... mechanical seal, 216b ... mechanical seal, 217 ... seal chamber, 218 ... communication nozzle, 219 ... liquid level indicator, 220 ... culture solution circulation channel, 221 ... culture solution circulation channel, 222 ... culture solution, 231 ... bioreactor/fermentor, 232 ... cell separation device, 233 ... medium vessel, 234 ... tank in analyzer, 235 ... drive motor, 236 ... mixer, 237 ... air diffuser, 238 ... measuring device, 239 ... measured value, 240 ... valve, 241 ... valve, 242 ... valve, 243 ... valve, 244 ... valve, 245 ... valve, 246 ... valve, 247 ... valve, 248 ... pump, 250a ... individual operation device for regulating gas supply, 250b ... individual operation device for regulating gas supply, 251 ... pressure gauge, 252 ... pressure control valve, 253 ... communication channel, 254 ... valve, 255 ... mixer, 256 ... mixer drive motor, 257 ... pressure gauge, 258 ... pressure control valve, 259 ... air supply valve, 260 ... valve, 261 ... mixer, 262 ... mixer drive motor, 263 ... pressure gauge, 264 ... pressure control valve, 265 ... air supply valve.

### DETAILED DESCRIPTION OF THE PREFERRED EMBODIMENTS

The system for cultivating cells of the present invention is hereinafter described in detail with reference to drawings. The system for cultivating cells of the present invention is applicable to cultivate cells which produce a substance to be a principle component of pharmaceuticals, etc. In the present invention, examples of the subject substance to be produced include proteins such as antibody, enzyme, etc., and bioactive substances such as low molecular compounds, high molecular compounds, etc. Examples of the cells to be cultivated include animal cell, plant cell, insect cell, bacteria, yeast, fungus and algae, etc. It is particularly preferable to use an animal cell which produces a protein such as antibody, enzyme, etc., as a subject for culture.

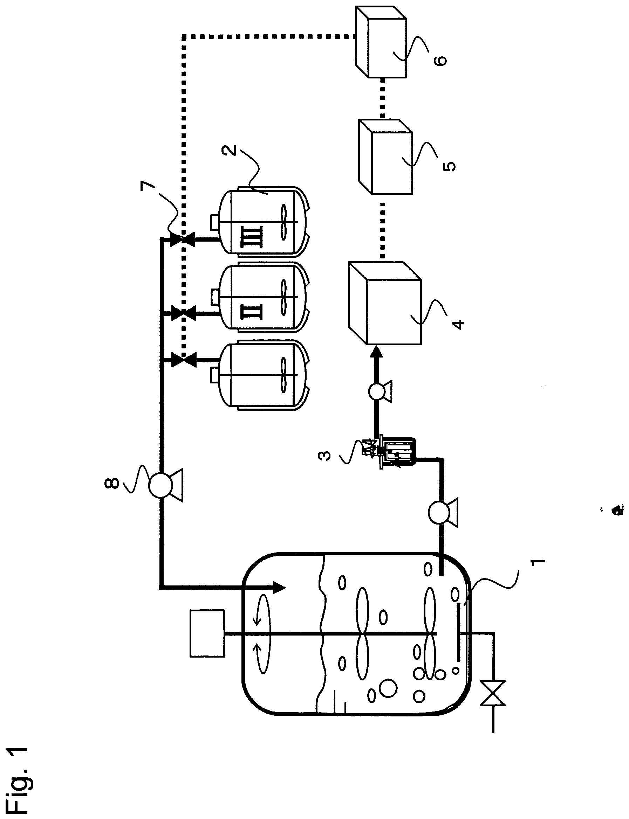

The system for cultivating cells of the present invention, as shown in FIG. 1, is constituted by a bioreactor/fermentor 1, a plurality of feed medium vessels 2, an aseptic sampling device 3, an analyzer 4, an analysis device 5, and a control device 6. The system for cultivating cells of the present invention preferably samples the culture solution aseptically from the bioreactor/fermentor 1 and measures the component in the culture solution, etc. Based on the measurement results, the state of a cell at the time of the measurement is analyzed. In the system for cultivating cells of the present invention, the control device 6 selects the optimal feed medium based on the state of the cell, determines an amount to be fed, and controls the selection of the feed medium to be added from a plurality of feed medium vessels 2 by activating a valve 7 and a pump 8. The selection of the feed medium in accordance with the state of a cell can significantly enhance the productivity of an intended substance, etc., produced by the cell.

Examples of the plurality of feed media to be loaded in a plurality of the feed medium vessels 2 herein include those designed to have different compositions of a carbon source and/or a nitrogen source among the feed media used for the typical fed-batch culture. Examples of the carbon source include glucose, sucrose, etc., and examples of the nitrogen source include amino acids such as glutamic acid, etc. An example is the plurality of feed media designed so that a carbon source concentration is constant and a nitrogen source concentration changes in steps. Alternatively, another example is the plurality of feed media designed so that a nitrogen source concentration is constant and a carbon source concentration changes in steps. Alternatively, another example is the plurality of feed media designed so that a carbon source concentration and a nitrogen source concentration respectively change in steps.

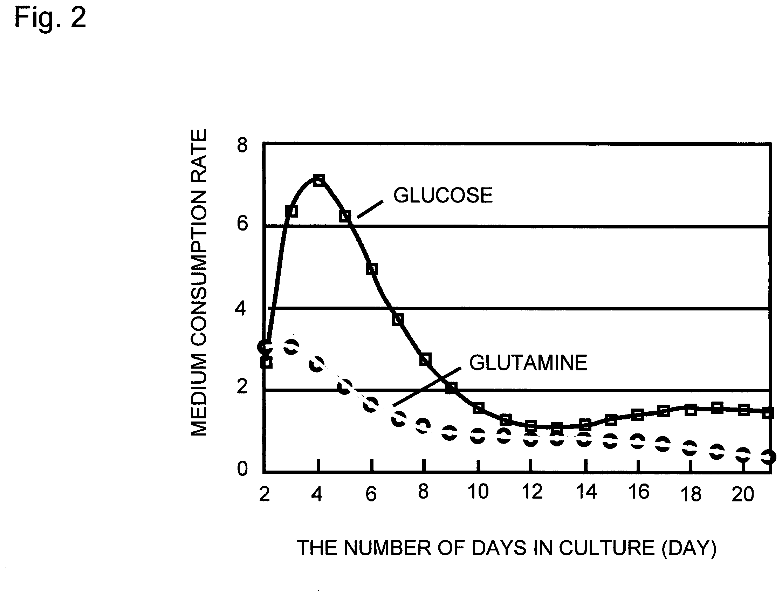

Indeed, it is documented that the IFN-gamma producing CHO cell increases the production of antibody when cultivated under maintained constant concentration by independently controlling a glutamine concentration and a glucose concentration (Chee Fumg Wong D, et al., Biotechnol Bioeng. 2005, Jan. 20; 89(2): 164-177.). Further, it is known that when a glutamine concentration in the culture solution or the ratio of a glutamine concentration to a glucose concentration changes, the glycosylation pattern which affects the antibody activity changes. More specifically, according to the above document, it is reported that the glycosylation pattern changes between when IFN-gamma producing CHO cell is cultivated at a glutamine concentration maintained at 0.1 mM, 0.3 mM and 0.5 mM, respectively, and when cultivated at a glutamine concentration maintained at 0.3 mM and a glucose concentration maintained at 0.35 mM and 0.7 mM, respectively. Accordingly, produced antibodies may not have uniform quality from lot to lot due to the nutritional imbalance in the culture solution. To solve this problem, it is preferable to control the concentrations of glutamine and glucose, respectively, within the range of ±0.1 mM and ±0.18 mM. According to the above document, when a hybridoma cell is subjected to the conventional fed-batch culture, the consumption rate ratio of glucose to glutamine remarkably changes with time as shown in FIG. 2, whereby the concentrations cannot be controlled within the above range throughout the entire culture process.

For the optimal control, a suitable approach must be taken for each of (I) analyzing to ascertain culture state, (II) optimizing the design of feed medium components, (III) controlling apoptosis inhibition, (IV) component analysis method, and (V) determining aseptic sampling. In the present invention, the best embodiment is achieved by employing the supposedly optimal approaches in each of (I) to (V), but is not limited to these method combinations, and effects can be attained by applying even at least one of these approaches. The best method in each procedure is hereinafter described.

### (I) Analysis to ascertain culture state

A metabolic flux analysis which analyzes the metabolism in a cell is used. Since the technique of the conventional intracellular metabolic flux analysis requires to regulate a state of cells until an intracellular metabolic state is reflected to the labeled information on an amino acid, time-dependent changes in an intracellular metabolic flux distribution could not be evaluated. Under this circumstance, time-dependent changes in an intracellular metabolic flux distribution can be measured from either one of the experiments of the following two methods.
(1) Targeting an intermediate metabolite which has a smaller pool size than amino acids constituting proteins and whose metabolic changes are quickly reflected to the label pattern, the time-dependent change in an intracelullar flux is measured by estimating a metabolic flux distribution from each labeled information. CE-TOFMS (capillary electrophoresis-time-of-flight mass spectrometry) can be used as a technique for directly measuring the labeled information of an intracellular intermediate metabolite. CE-TOFMS does not require cumbersome pretreatments, simultaneously determines a number of ionic low-molecular compounds from a small amount of samples, and estimates a metabolic flux distribution.
(2) Isotopically labeled glucose is taken into culture, and glucose, glycerol, acetate, citrate and pyruvate are analyzed using NMR, HPLC and GC/MS. The analysis data is analyzed for an intracellular metabolic flux distribution in an unsteady state, using Elementary Metabolite Unit (EMU) (Antoniewicz MR, et al., Metab. Eng. 2007, Jan.; 9(1): 68-86).

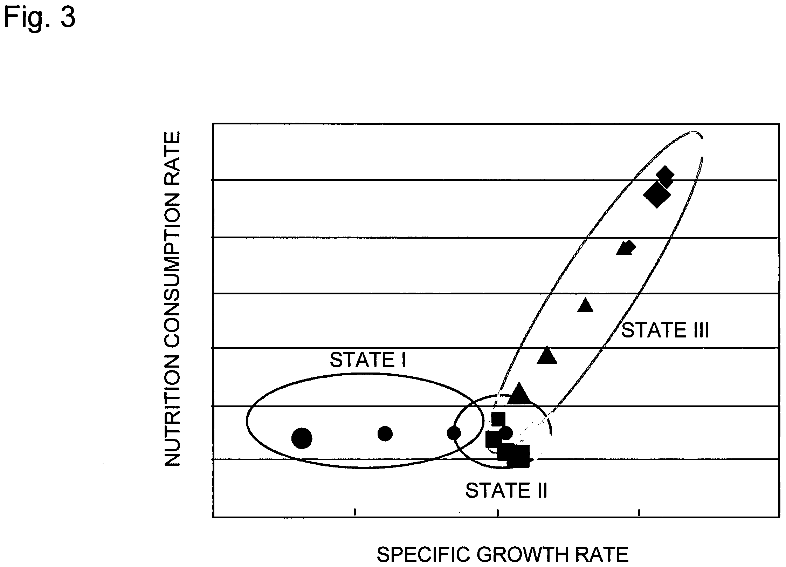

Given is an example in which the time-dependent changes in the nutrient concentration of culture cells were analyzed using the metabolic flux analysis, and the analyzed results were applied to the classification of the culture state. FIG. 3 shows the relationship between the consumption rate and specific growth rate of glucose and glutamine. In this case, the state can be classified into three states as shown in the figure. The cell state is classified by measuring the growth rate during culture, thereby feeding a feed medium suitable for each state. The present invention shows that the culture state can be classified using the specific growth rate as an indicator, but the classification can be made using other indicators.

### (II) Optimized design of feed medium components

For designing a feed medium suitable for a culture state, it is effective and desirable to design the medium in accordance with a metabolic flux analysis in an unsteady state as used in the analysis to ascertain the culture state in the above (I).

When a metabolic flux distribution in an unsteady state is determined by the method described in (I), a metabolic rate of amino acid, etc., at each state of culture cells can be determined. The thus determined metabolic rate is the bare minimum consumption required for cells to sustain the life and grow, and a nutrient amount exceeding that consumption invites an unnecessary increase of a secretion. The optimal nutritional environment for cell growth can be achieved by considering such a metabolic rate as a consumption to be consumed by cells. A feed medium composition ratio is determined based on a metabolic flux distribution (an averaged value of the entire culture processes) also in the conventional method (Xie L and Wang DI 1994, Cytotechnology 15(1-3): 17-29). In the optimized design of the present invention, a metabolic rate of each amino acid, etc., at each culture state is determined by employing the same technique to a metabolic flux distribution of each culture state (each culture time) and a feed medium suitable for each state of culture cells can be designed by making feed medium components as a ratio of each nutrition metabolic rate.

It is documented that the analysis of time-dependent changes in a metabolic flux distribution by the metabolic flux analysis in an unsteady state has an accuracy of 10% error in *E. coli* (Antoniewicz MR et al., Metab Eng 2007 May; 9(3)277-292). Accordingly, the medium design of the present invention can be designed with 10% error. In the typical fed-batch culture control, the culture is often carried out by regulating the concentrations of glutamine and glucose at 0.3 mM and 0.4 mM, with the limits of error being 0.03 mM and 0.04 mM, respectively. Consequently, it is possible to regulate the to-be-attained concentrations of glutamine and glucose at ±0.1 mM and ±0.18 mM, respectively.

As for the feed medium, it is not limited to those described above, and, for example, the regulation may be achieved by independently designing glucose and glutamine to effectively inhibit ammonia and lactic acid secretions, and feeding them independently in accordance with a culture state.

### (III) Controlling apoptosis inhibition

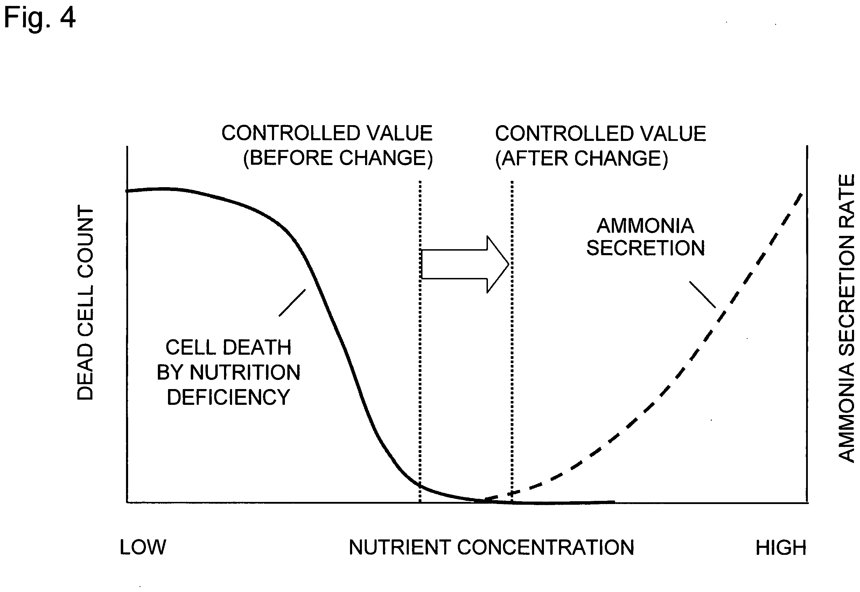

As shown in FIG. 4, the culture is performed while maintaining nutrient concentrations such as amino acids, etc., low to inhibit the ammonia accumulation. However, if a part of the nutritional components becomes insufficient in concentration from the nutrient consumption by a cell, apoptosis is induced. The tolerance against apoptosis from the nutritional deficiency (apoptosis resistance) varies even among allogeneic cells, and it is reported that such a resistance depends on mitochondrion membrane potential in a cell. Apoptosis by undernutrition is first induced in cells with a low apoptosis resistance, and then spreads to those with a higher apoptosis resistance. Under such a circumstance, a control is achieved wherein apoptosis is monitored during the culture steps and the controlled value of nutrient concentration is set to a higher value at the time of detecting apoptosis.

### (IV) Component analysis method and system

In performing supply control of a feed medium, examples of the indication to be monitored include viable cell count and apoptosis cell count. To ascertain a culture state from the culture solution which does not contain cells, the following detection method is applicable.

### Viable cell count

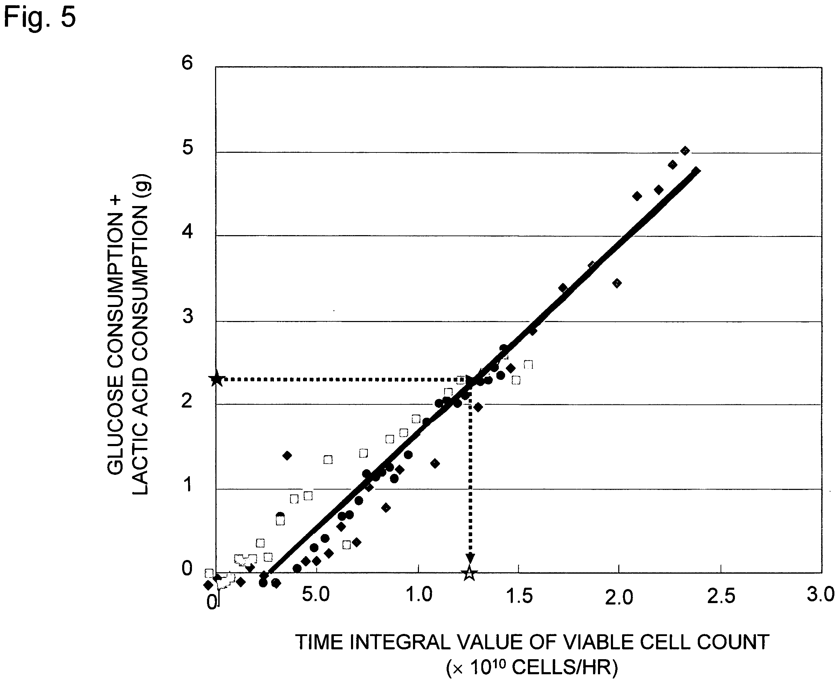

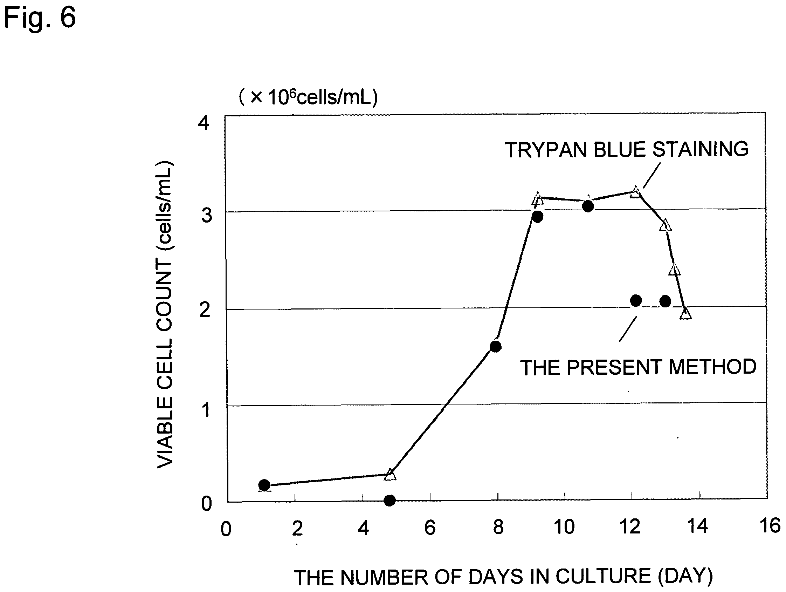

A method can be used wherein a viable cell count is estimated by measuring an amount of glucose and an amount of lactic acid in a culture solution. As shown in FIG. 5, the sum of glucose consumption and lactic acid consumption is in the linear relationship with the time integral value of the viable cell count. With this relational expression being measured beforehand as a calibration curve, a viable cell count during the culture steps can be estimated by determining the sum of glucose consumption and lactic acid consumption by the measurement. The comparison between the viable cell count estimated by this method and the viable cell count estimated by the conventional trypan blue staining is as shown in FIG. 6 which shows both counts are well synchronized. From this result, a viable cell count can be measured by this method.

### Apoptosis

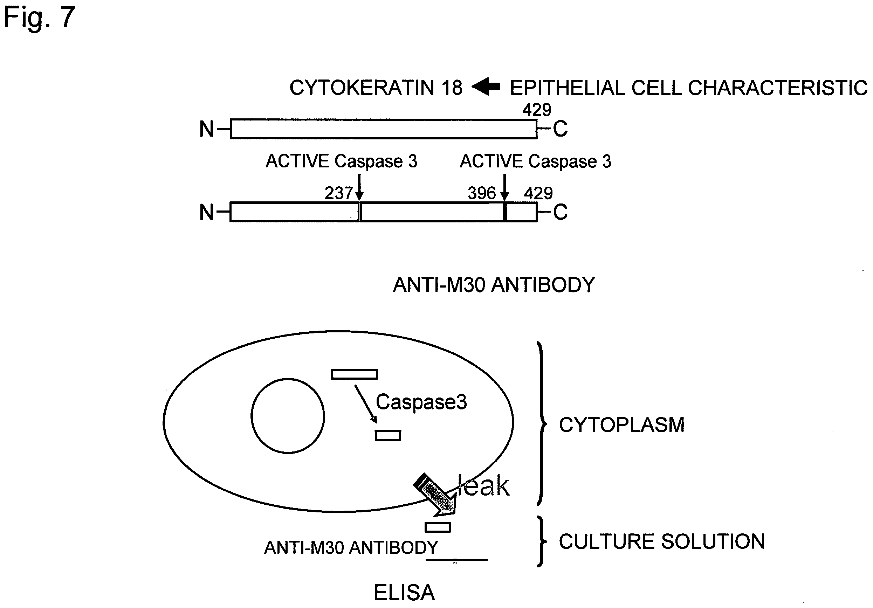

Since cytokeratin 18 protein expressed in a cell is cleaved by caspase 3 resulting from apoptosis and secretes fragments (M30 fragment) having a M30 recognition site, a method can be employed wherein the fragment secreted in the culture solution can be detected by ELISA using the M30 antibody (FIG. 7). Shown below is an example in which apoptosis was measured by the ELISA method using a 96-well plate.

The ELISA method using the M30 antibody is first described. Anti-cytokeratin 18 antibody (M5 antibody; Roche Diagnostics) was bonded to a 96-well plate. Blocking was then carried out using skim milk, a culture solution was fed thereto, and an incubation was conducted at 37°C for an hour. After washing three times, a biotin-labeled M30 antibody solution (Roche Diagnostics) was added thereto, and an incubation was conducted at 37°C for an hour. After washing three times, an HRP-labeled Streptavidin solution was added thereto, and an incubation was conducted at 37°C for an hour. After washing three times, a TMB substrate solution was added thereto, an incubation was performed at room temperature for 20 minutes, and subsequently sulfuric acid was added, thereby measuring an absorbance at 450 nm.

As a specific example, apoptosis was measured using a culture solution in which a CRL-1606 cell was fed-batch cultivated in a 1-L bioreactor/fermentor. The culture with stirring was carried out under the culture conditions of 37°C and a 60% saturated dissolved oxygen concentration. 5 mL of the culture solution aseptically containing cells was sampled every 12 hours, and 1 mL of this solution was subjected to measurement of the total cell count and viable cell count, using a ViCell viable cell counter (Beckman Coulter). The cells in the remaining culture solution were settled using a centrifuge (800 rpm, 5 minutes), and the supernatant was cryopreserved. After completing the culture, the M30 fragment was detected in the same manner as above.

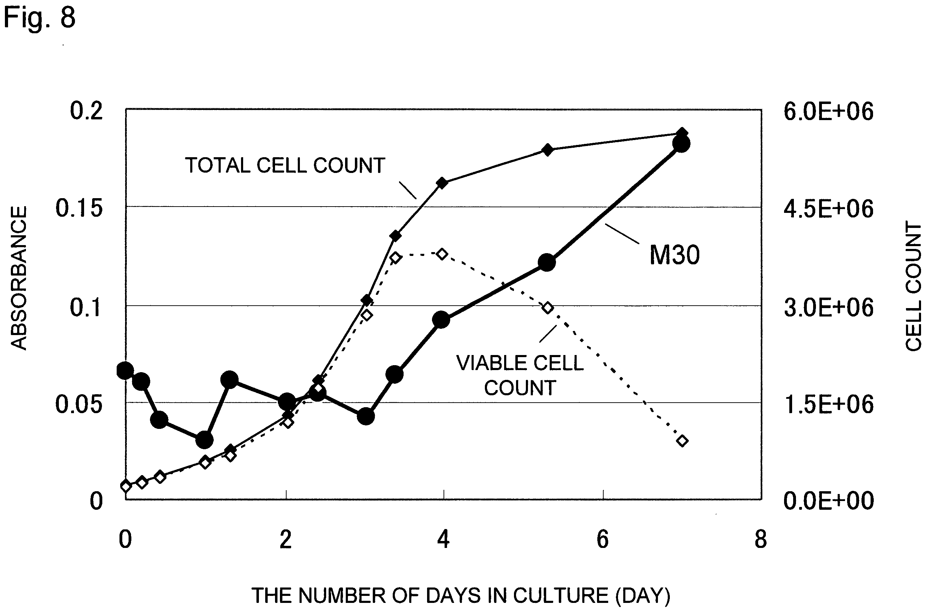

FIG. 8 is a graph showing the time-dependent changes in the M30 fragment in the culture solution in the 1 L bioreactor/fermentor and the time-dependent changes in the total cell count and viable cell count. As the viable cell count decreases, i.e., as the dead cell count increases, the amount of M30 fragment is found increased. It is verified that the method for detecting apoptosis of the present invention enables the detection of apoptosis from the culture solution.

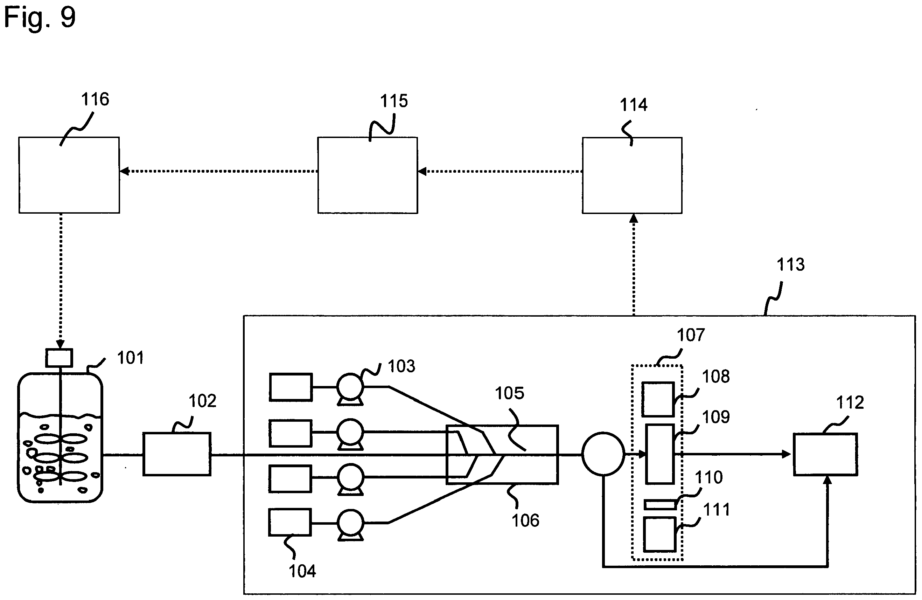

The viable cell count and apoptosis can be measured from the culture solution by the method described above, but apoptosis may also be measured using a microreaction field for even quicker measurement with higher sensitivity. A system for cultivating cells equipped with a microreaction field has the structure shown in FIG. 9. More specifically, the system for cultivating cells is provided with a bioreactor/fermentor 101, a filter 102 for separating cells contained in the culture solution in the bioreactor/fermentor 101, an apoptosis detector 113 for detecting the M30 fragment contained in the culture solution free of the cells via the filter 102, a recorder 114 for recording measured values resulting from the M30 fragment detected by the apoptosis detector 113, an analysis device 115 for analyzing the apoptosis based on the measured values recorded by the recorder 114, and a control device 116 for selecting a feed medium to be added to the bioreactor/fermentor 101 out of a plurality of feed-medium vessels (not shown) based on the results analyzed by the analysis device 115. Further, the apoptosis detector 113 is provided with pumps 103 for fluxing a reagent required for each component analysis to be described later, syringes 104 for containing the reagents, a microchannel 105 for detecting the M30 fragment, a chip 106 on which the microchannel 105 is formed, an absorption spectrometer 107, and a waste fluid tank 112. Furthermore, the absorption spectrometer 107 consists of a detector 108, a cell 109, and a wavelength filter 110 arranged on the optical axis from a light source 111.

In the thus structured system for cultivating cells, a primary antibody (anti-cytokeratin 18 antibody; M5 antibody) is allowed to bond in the microchannel 105. The culture solution, passed through the filter 102 to prevent the cells from contaminating from the bioreactor/fermentor 101, is allowed to flow into the apoptosis detector 113 using a pump not shown. If the cell death is apoptosis, the M30 fragment is present in the culture solution and binds to the above primary antibody. The biotin-bonded M30 antibody is then fluxed using a microsyringe. After fluxing a wash solution, streptavidin conjugated with alkaline phosphatase is allowed to flow. After fluxing the wash solution, NBT (nitro-blue tetrazolium chloride) substrate is delivered, and an absorbance was measured at 405 nm using the absorption spectrometer 107, thereby measuring the presence or absence and an amount of apoptosis. At the completion of the measurement, the proteins excluding the primary antibody can be washed out by flowing an acidic solution or alkaline solution through the channel. Another apoptosis measurement can be carried out using the same chip 106 by fluxing a wash solution and delivering a blocking solution. The measured results are recorded by the recorder 114 and analyzed by the analysis device 115 to attain optimal control, and a not shown plurality of feed-medium vessels are controlled by the control device 116, whereby culture can be performed under the optimal environment for cells and the productivity is hence enhanced. Further, the data stored in the recorder 114 can be used as one of the quality control indicators.

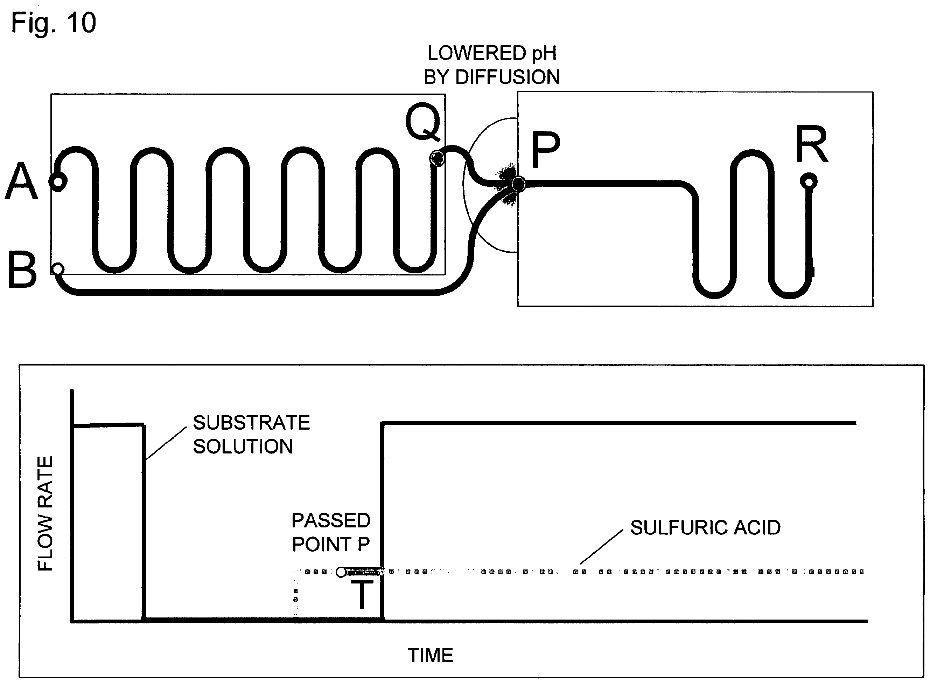

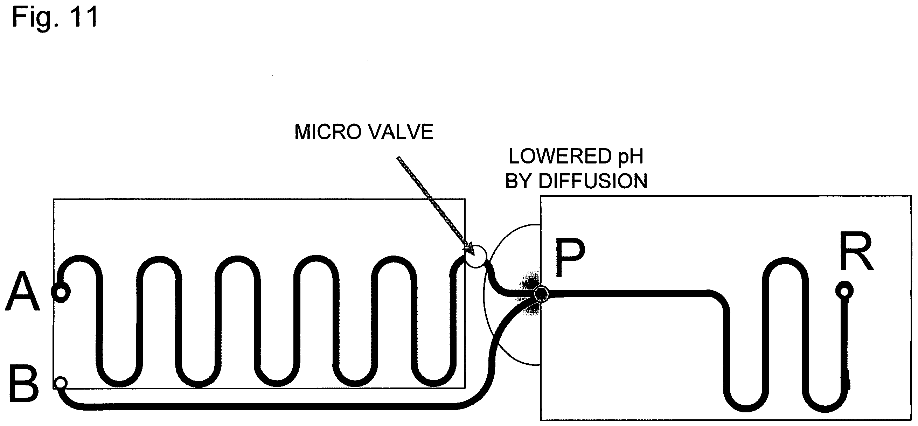

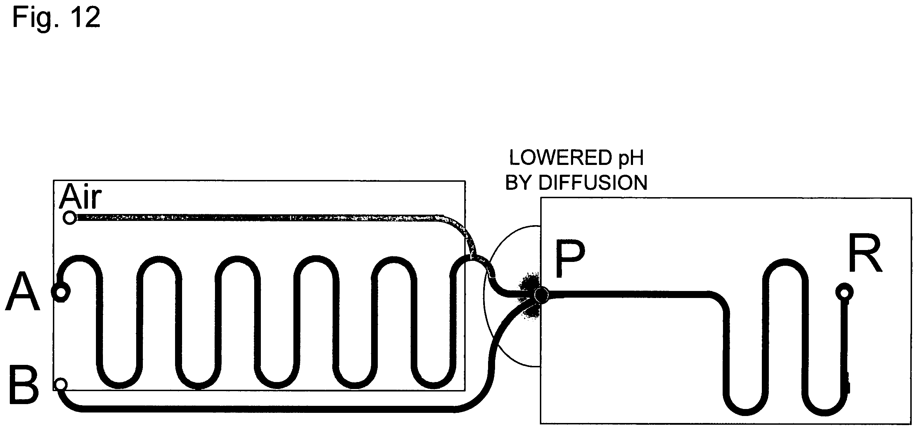

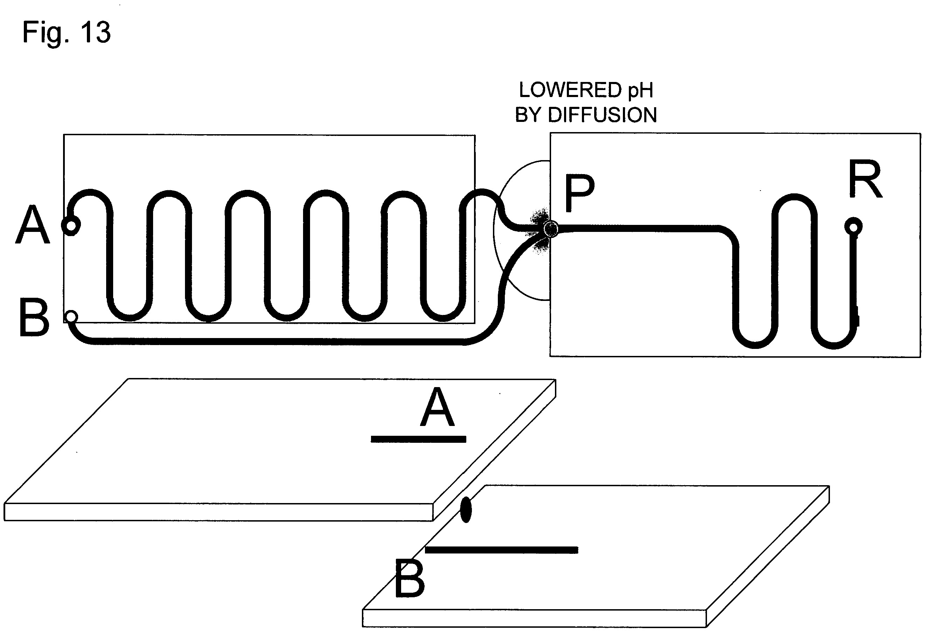

The streptavidin conjugated with alkaline phosphatase was used in the above, but streptavidin conjugated with horseradish peroxidase may also be used. When using streptavidin conjugated with horseradish peroxidase, TMB (3,3',5,5'-tetramethylbenzidine) is used as a chromogenic substrate. After the reaction, sulfuric acid is mixed therein and an absorbance at 450 nm is measured. In this case, a microreaction field consists of, for example, an antibody reaction unit as indicated between A and Q, a sulfuric acid flow channel as indicated between B and P, and a mixing channel for mixing the sulfuric acid and the reacted TMB as indicated between P and R, as shown in FIG. 10. After loading the unit between A and Q with TMB for an antibody reaction, the flow is withheld to secure a sufficient reaction time. After the adequate reaction, the flow is resumed and mixed with sulfuric acid. At the time of resuming the flow, sulfuric acid may flow into the reaction field from the sulfuric acid flow channel (including the inflow by the diffusion). Thus, if the mixing channel becomes an acidic condition, the physical bonds between proteins are cleaved whereby the precise measurement may not be achieved. To improve the possible problem, the channel structure should be either one of the following structures.
(1) As shown in FIG. 10, sulfuric acid is fluxed to satisfy T<τ_{PQ} (τ_{PQ}: time required for pH to be lowered at point Q by the diffusion of the withheld solution).
(2) As shown in FIG. 11, a micro valve is provided between a microchannel and sulfuric acid flow channel. The valve is closed while the reaction is proceeding to separate the solution flow channel from the reaction-quenched solution channel, and the valve is opened during the detection to mix sulfuric acid with the reaction-quenched solution.
(3) As shown in FIG. 12, an air supply channel is provided between the microchannel and sulfuric acid flow channel to separate sulfuring acid from the reaction-quenched solution by supplying an air, thereby separating the reaction solution.
(4) As shown in FIG. 13, a difference in height is provided between the microchannel and sulfuric acid flow channel to separate the reaction-quenched solution channel from the reaction solution.

The above structures (1) to (4) enable the prevention of sulfuric acid from flowing (including the inflow by the diffusion) into the microchannel for the reaction.

Further, the chip 106 to which the primary antibody is bound has an amino group treated on the channel inner wall. First, a glutaraldehyde solution is allowed to flow through the channel to activate the amino group, the channel is washed with pure water, and M5 antibody is delivered to be covalently bound to the channel inner wall. After washing, the blocking solution is fluxed to close the channel opening to prevent it from drying, thereby making it possible to store at 4°C. Since the chip, once mounted to the apoptosis detector before the operation of the bioreactor/fermentor, can be reused, it does not need to be replaced until the operation is completed. The method for covalently binding the antibody to the chip channel may be performed using a carboxyl group. The binding using the covalent bond is a desirable method when the chip is reused, but the binding using the hydrophobic bond may be employable when the chip does not need to be reused.

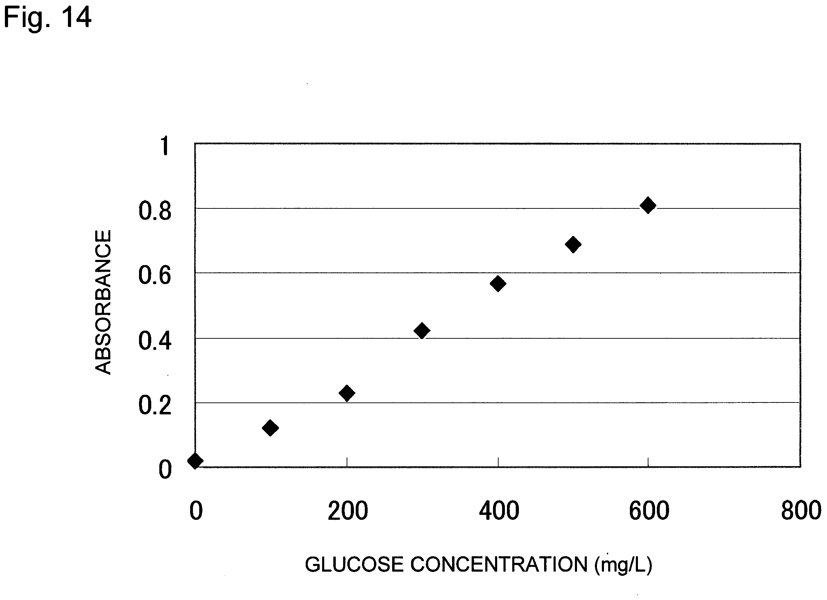

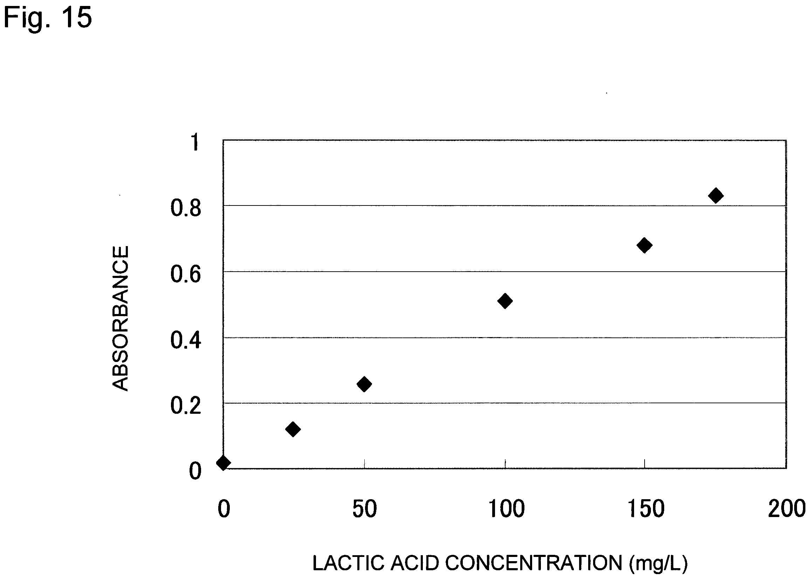

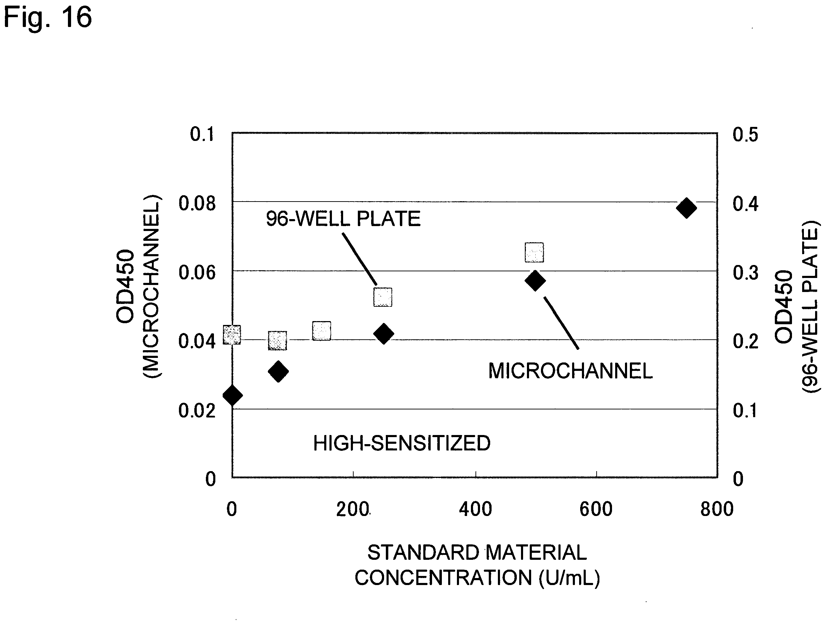

The experiment results obtained using the system for cultivating cells with the above microreaction filed are described hereinafter. FIG. 14 shows the relationship between the glucose concentration and chromogenic intensity. In the method, the figure shows a linear relationship up to a glucose concentration of 600 mg/1. FIG. 15 shows the relationship between the lactic acid concentration and chromogenic intensity. In the method, the figure shows a linear relationship up to a lactic acid concentration of 135 mg/1. FIG. 16 shows the relationship between apoptosis and chromogenic intensity. The measurement can be carried out with higher sensitivity when a microreaction field is used than when a 96-well plate is used.

For the glucose detection, a colorimetric assay was employed wherein the hydrogen peroxide, produced when glucose is oxidized by an enzymatic reaction with glucose oxidase, was caused to be produced, and the hydrogen peroxide, p-hydroxybenzoic acid amino and amino antipyrine were caused to form a red quinone imine compound by an enzymatic reaction with peroxidase. The mixing ratio of the chromogenic reagent and sample solution in the solution flux mixing reaction using the microchip was 9:1. A chromogenic reagent and a reagent were instilled at 90 µl/min and 10 µl/min, respectively, using a syringe pump and mixed in the chip, and an absorbance at a wavelength of 510 nm was measured using a spectrophotometer. The absorbance by the chromogenic reaction was determined by the variation between the thus obtained absorbance and the chromogenic solution absorbance that had been measured separately beforehand.

For the lactic acid detection, a colorimetric assay was employed wherein the hydrogen peroxide, produced when lactic acid is oxidized by an enzymatic reaction, was used. The mixing ratio of the chromogenic reagent and sample solution in the solution flux mixing reaction using the microchip was 9:1. A chromogenic reagent and a reagent were instilled at 90 µl/min and 10 µl/min, respectively, using a syringe pump and mixed in the chip, and an absorbance at a wavelength of 510 nm was measured using a spectrophotometer. The absorbance by the chromogenic reaction was determined by the variation between the thus obtained absorbance and the chromogenic solution absorbance that had been measured separately beforehand.

### (V) Aseptic sampling

To use the component analysis method described above, an aseptic sampling of the culture solution from which the cell has been removed is desirable. Below is an embodiment of the aseptic sampling apparatus of the culture solution for performing such an aseptic sampling.

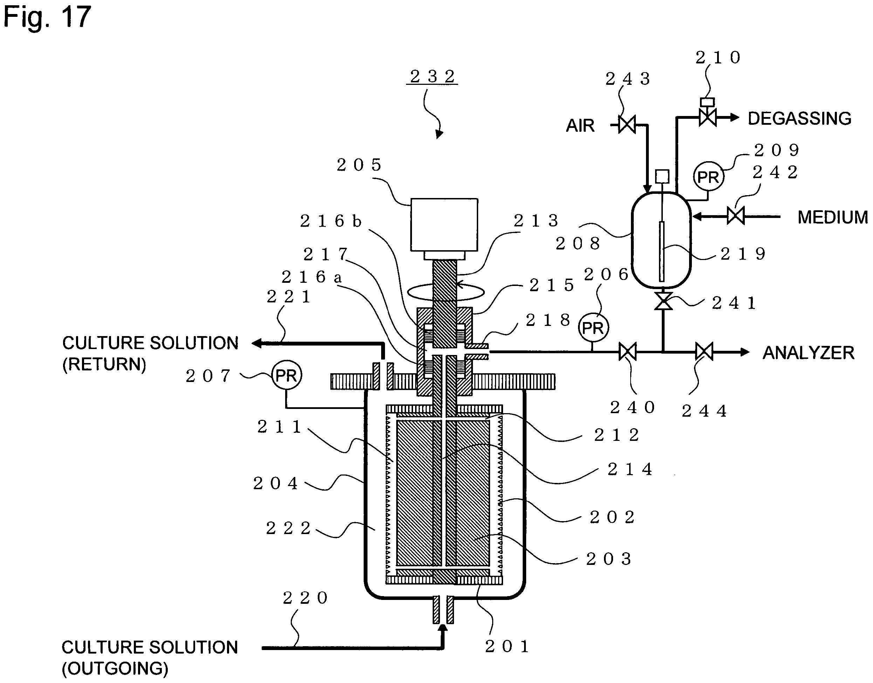

FIG. 17 is a flow diagram showing the operation of an embodiment of the aseptic sampling apparatus of the present invention. The sampling apparatus is provided with a rotating filter 201 equipped with a filter 202 on the circumferential surface for blocking the cell from passing therethrough, a filter container 204 for containing the rotating filter 201, a filter drive motor 205 for rotating the filter, a pressure gauge 206 for detecting the inner pressure of the rotating filter 201, a pressure gauge 207 for detecting the pressure of the filter container, a backwash water reservoir 208, a pressure gauge 209 for detecting the inner pressure of the backwash water reservoir 208, and a pressure control valve 210 for adjusting the pressure inside the backwash water reservoir 208.

The filter container 204 is provided with culture solution circulation channels 220 and 221 between the bioreactor/fermentor and itself for circulating the culture solution using a solution flow device such as a pump, etc. The bioreactor/fermentor and solution flow device are not shown in FIG. 17. Owing to this structure, the culture solution inside the filter container 204 is constantly replaced, thereby inhibiting the adverse affects to the culture resulted from the deteriorated environment caused by the partial high density of the cell due to draining of a filtrate by filtering and a prolonged retention time.

It is essential for the filter container 204 to have the function for preventing the culture solution inside from outflowing therefrom and blocking outside contaminants from entering the container. To impart these functions, the filter container 204 is provided with an axial seal 215 using mechanical seals. The mechanical seals serve to stop the leaks of gas and liquid by allowing a sliding member fixed to a rotation axis 213 to rotate and the other sliding member fixed to the axial seal 215 to tightly contact and slide together. The present embodiment uses two mechanical seals, i.e., a mechanical seal 216a for preventing the culture solution inside the filter container 204 from outflowing therefrom and the other mechanical seal 216b for blocking outside contaminants from entering the container. A seal chamber 217 is provided between the mechanical seals 216a and 216b to serve as a channel connecting the inside and outside the rotating filter 201. In the present invention, the mechanical seal is not limited, and can be any mechanical seals and dry mechanical seals used for the typical culture apparatus insofar as they are capable of maintaining airtightness. Further, the method for using and arrangement of the mechanical seals are not limited to those described in the present embodiment.

The rotation axis 213 has a hollow structure with both ends sealed, and forms an axial channel 214 by opening one end to the seal chamber 217 and opening the other end so that it communicates with a filtrate channel 212 provided in a cylinder 203 to be described later. The axial seal 215 is provided with a communication nozzle 218 at the position of the seal chamber 217, and used for taking in and out of a filtrate and backwash solution.

The rotating filter 201 has a structure wherein the cylinder 203 and filter 202 are mounted to the rotation axis 213 and the upper and bottom ends thereof are sealed with sealing members, wherein the filtration is carried out by lowering the pressure inside the filter than that of the outside, thereby obtaining the filtrate that has passed through pores of the filter. During the filtration, rotating the rotating filter 201 inside the filter container 204 causes parallel streams of the culture solution on the surface of filter 202, resulting in a so-called crossflow filtration state. When the gap between the inner wall of the filter container 204 and the surface of the filter 202 is made small to create a turbulent flow state, the highly densified layer of cells and microscopic particles formed by draining the filtrate can be diffused. Thus, clogging of a filter can be controlled, enabling acquisition of the filtrate in a larger amount with a higher speed. The rotation speed of the rotating filter is determined based on the resistance of an animal cell to be cultivated against physical external forces and the configuration of a separation device.

The filtrate passed through the filter 202 flows through a cylindrical gap 211 formed between the cylinder 203 and the filter 202, leads to the seal chamber 217 in the axial seal 215 via the axial channel 214 provided in the rotation axis 213 and the filtrate channel 212 provided in the cylinder 203, and is transferred to the backwash water reservoir 208 by way of valves 240 and 241 along the communication channel connected to the communication nozzle 218.

The filter used in the present system for cultivating cells is not limited as long as it can block the cell passage such as clothes, membrane filters, and like those typically used. In particular, it is preferable to select a filter having mechanical strengths, heat resistance and corrosion resistance to endure a washing solution injection when a steam is blown into the rotating filter for the sterilization and washing. Further, since numerous small cell fragments resulted from the cytolysis of dead cells are present in the culture solution, it is particularly preferable to use a filter having filtering properties which prevents healthy cells from passing therethrough and allows small cell fragments to pass therethrough. The present embodiment used a metal filter on which a slit opening was formed by cylindrically winding a stainless thread at given intervals. The typical filter having many pores smaller than the particle diameter to be blocked even blocks micro cell fragments, thereby causing the clogging. There is no pore substantially existing in the filter used in the present embodiment, other than the slit deliberately made. Thus, the present filter prevents the only cells larger than the slit width from passing therethrough and allows the microparticles such as cell fragments, etc., smaller than the slit width to pass therethrough. The slit width is determined in accordance with the size of cell to be cultivated, and typically ranges from 5 to 30 µm.

The backwash water reservoir 208 is provided with a liquid level indicator 219 housed therein for measuring a liquid level, a pressure gauge 209 for detecting an internal pressure, and a pressure control valve 210 for adjusting the inner pressure of the backwash water reservoir. The backwash water reservoir 208 is connected to the communication nozzle 218 by opening the valves 241 and 244, a tank 234 in the analyzer by opening valves 241 and 244, and the bioreactor/fermentor by opening a valve 242, respectively, thereby enabling the solution to travel. The tank 234 in the analyzer and the bioreactor/fermentor are not shown in FIG. 17.

The pressure adjustment inside the backwash water reservoir 208 is carried out by adjusting an air amount blown thereinto by opening and closing the valve 243 and adjusting a degassing amount by opening and closing the pressure control valve 210. The inner pressure of the backwash water reservoir 208 is constantly maintained higher than atmospheric pressure in the present invention. The air used for adjusting the pressure is aseptic air from which bacteria and like microparticles have been already removed.

The liquid level indicator 219 is not limited, but it is preferable to select those having mechanical strengths, heat resistance and corrosion resistance to endure a washing solution injection when a steam is blown into the rotating filter for the sterilization and washing. Further, since numerous cells and small cell fragments resulted from the cytolysis of dead cells are present in the culture solution and bubbles are likely to stay on the liquid surface, it is preferable to select those less likely to cause malfunctions due to the dirty sensor. The electrostatic capacity level sensor was used in the present embodiment.

The separation technique of an animal cell using the present embodiment is hereinafter described in detail.

### (1) Filtration step

The culture solution is allowed to circulate in the filter container 204, and the rotating filter 201 is rotated using the filter drive motor 205. The pressure of the filter container was measured using the pressure gauge 207, and the pressure of inside the backwash water reservoir 208 was measured using the pressure gauge 209. The pressure of the filter container is the same as that of the bioreactor/fermentor, and is typically pressurized to 0.01 to 0.05 MPa. The pressure inside the backwash water reservoir 208 is adjusted using the pressure control valve 210 so that it is slightly lower than the pressure of the filter container. Consequently, the valves 241 and 240 are opened one by one, the filtration is performed in the filter 202 and the filtrate flows into the backwash water reservoir 208. The liquid level of the filtrate is measured using the liquid level indicator 219, and the valves 240 and 241 are closed one by one at the time of the level reaching a determined level, whereby the filtration step is completed. It is not preferable to carry out the filtration operation rapidly by applying high filtration differential pressure because it facilitates the clogging. In the present embodiment, the differential pressure of the filter container 204 and the backwash water reservoir 208, i.e., the filtration differential pressure, is set to 0.04 MPa or lower. The adjustment of the filtration rate is performed by adjusting the filtration differential pressure of the filter container 204 and the backwash water reservoir 208. When the pressure needs to be kept lower than the filtration rate, the open time duration is adjusted by continuously opening and closing the valve 240 to attain a desired pressure.

### (2) Filtrate discharge step

The inner pressure of the backwash water reservoir 208 is set higher than that of the tank 234 provided in the analyzer by opening the valve 243. Subsequently, the filtrate is delivered to the tank 234 in the analyzer by opening the valves 241 and 244 one by one. The liquid level of the filtrate is measured using the liquid level indicator 219, and the valves 244 and 241 are closed one by one at the time of the level reaching a determined level, whereby the filtrate discharge step is completed. For setting the pressure, it is necessary to consider the liquid levels of the tank 234 in the analyzer and the backwash water reservoir 208. More specifically, when the liquid level of the tank 234 in the analyzer is higher than that of the backwash water reservoir 208, the pressure must be set high not to cause a back flow. When the liquid level of the tank 234 in the analyzer is lower than that of the backwash water reservoir 208, air is injected so as not for the inner pressure of the backwash water reservoir 208 to become lower than atmospheric pressure due to the filtrate outflow.

### (3) Medium delivery step

The inner pressure of the backwash water reservoir 208 is adjusted to be lower than that of the bioreactor/fermentor using the pressure control valve 210. Subsequently, the medium flows into the backwash water reservoir 208 by opening the valve 242. The liquid level of the medium is measured using the liquid level indicator 219, and the valve 242 is closed at the time of the level reaching a determined level, whereby the medium supply is completed. For setting the pressure, it is necessary to consider the liquid levels of the bioreactor/fermentor and the backwash water reservoir 208. More specifically, when the liquid level of the bioreactor/fermentor is higher than that of the backwash water reservoir 208, the differential pressure must be set low so as not for the delivery rate of the medium to be high. When the liquid level of the bioreactor/fermentor is lower than that of the backwash water reservoir 208, the differential pressure must be set high so that the medium is delivered. When the delivery rate of the medium needs to be adjusted, the open time duration is adjusted by intermittently opening and closing the valve 242 for the desired adjustment.

### (4) Backwash step

The inner pressure of the backwash water reservoir 208 is set higher than that of the filter container 204 by opening the valve 243. The number of rotations of the rotating filter 201 is increased greater than that in the filtration step. Subsequently, a medium is injected to the rotating filter 201 by opening valves 241 and 240 one by one, allowed to pass through the pores of the filter 202 backward from the direction in the filtration step, flows into the filter container 204, and is supplied to the bioreactor/fermentor by way of the culture solution circulation channel 221. More specifically, the medium, when supplied to the bioreactor/fermentor, serves as a backwash solution of the filter 202.

The liquid level of the medium is measured using the liquid level indicator 219. At the final stage of the backwash step when the determined level is attained, the inner pressure of the backwash water reservoir 208 is adjusted, using a pressure control valve 210, to be higher than the inner pressure of the filter container 204 and lower than the pressure combining the inner pressure of the filter container 204 and the bubble point pressure of the filter 202. Subsequently, after the predetermined time has lapsed, the valves 244 and 241 are closed one by one, and the number of rotations of the rotating filter 201 is decreased to the same as in the filtration step, thereby completing the backwash step.

The backwash, carried out in the rotating filter 201 spun at a high speed, enables the backwash solution to spread uniformly throughout the entire inner surface of the filter 202. Further, the cleaning effect additionally provided by the centrifugal force generated from the rotation imparts even greater backwash effects. When the inner pressure of the backwash water reservoir 208 is adjusted to be higher than the inner pressure of the filter container 204 and lower than the pressure combining the inner pressure of the filter container 204 and the bubble point pressure of the filter 202, the medium in the cylindrical gap 211, filtrate channel 212, axial channel 214 and communication channel can be all discharged to the filter container 204. In other words, the expensive medium can be entirely subjected to the culture with no waste.

The bubble point pressure of the filter is determined by sinking the rotating filter 201 in the medium, injecting an air therein, and measuring the pressure at the time of bubbles first being released from the filter surface.

In the embodiment which is to be the present invention, the separation operation of animal cells can be continued by repeating the above operations (1) to (4).

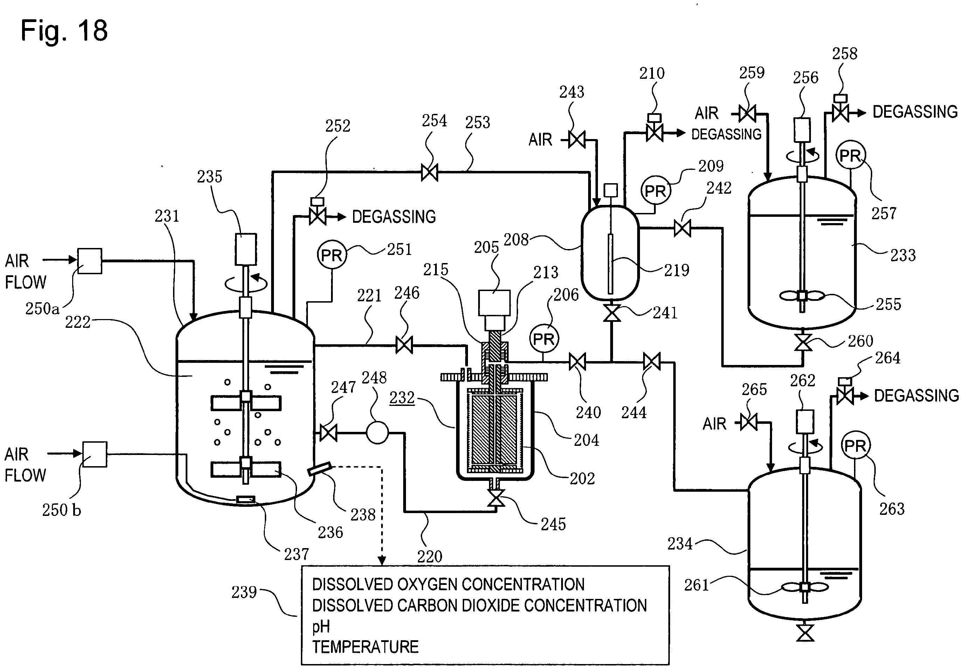

FIG. 18 is a flow diagram depicting the operation of an example of the system for cultivating cells according to an embodiment of the present invention. The present system for cultivating cells consists of a bioreactor/fermentor 231, a cell separation device 232, medium vessels 233 storing a feed medium and a tank 234 in the analyzer. The system for cultivating cells is provided with a plurality of medium vessels 233, but it is not shown in FIG. 18. Further, as the cell separation device 232, the separation device of the animal cell of the present invention shown in FIG. 17 is used. The system for cultivating cells is provided with, but not shown in FIG. 18, a system for supplying gas such as air, oxygen, nitrogen, carbon dioxide, etc., a hot/cold water supply system, a steam supply system and a plumbing system, which are all essential.

The bioreactor/fermentor 231 is shown in a cross sectional view. The culture solution 222 placed in the bioreactor/fermentor 231 is stirred by a mixer 236 powered by a drive motor 235 and mixed homogeneously. Oxygen required for the culture is supplied by two methods; the in-solution gas-flow technique wherein an oxygen-containing gas is supplied into the solution from the air diffuser 237 disposed on the bottom of the bioreactor/fermentor, and the upper surface gas-flow technique wherein the gas is aerated into the gaseous phase portion at the upper part of the bioreactor/fermentor 231.

The bioreactor/fermentor 231 is provided with a measuring device 238 for measuring characteristics of the culture solution 222 and obtains the measured values 239 of dissolved oxygen concentration, dissolved carbon dioxide concentration, pH, temperature, ammonia concentration, lactic acid concentration, glucose concentration, glutamine concentration and caspase or caspase digest concentration. As for the measuring device 238, the actual system is provided with a detection device at each item to be detected or controlled, but FIG. 18 shows only one device for simplicity. The measured values 239 are input into the not shown control device and used as the information for selecting a feed medium from a plurality of medium vessels 233 as described earlier.

The gas-flow systems to the solution and the upper surface are provided with individual operation devices 250a and 250b, respectively, for regulating the gas supplies. Further, the individual operation devices 250a and 250b are each provided with a flow rate control function and a supply measurement function for each of air, oxygen and carbon dioxide. For the gas flow to the upper surface, the individual operation device 250a which controls the gas supply regulates the gas composition and gas flow rate. In the present embodiment, air was flowed at a constant rate, and carbon dioxide was mixed in accordance with pH of the culture solution. The regulation of a carbon dioxide concentration was carried out, using pH as controlled variable, by the typical proportional control wherein the carbon dioxide flow rate is the operation factor. For the gas flow to the culture solution by the air diffuser 237, the individual operation device 250b which controls the gas supply regulates the gas composition and gas flow rate. In the present embodiment, the dissolved oxygen concentration of the culture solution is a controlled variable, and the oxygen flow rate is the operation factor.

The bioreactor/fermentor 231 has a constant pressure maintained by the pressure control valve 252, based on the measurement results by the pressure gauge 251. The pressure is typically applied to 0.01 to 0.05 MPa to prevent bacteria, etc., from entering the bioreactor/fermentor from outside.

Culture solution circulation channels 220 and 221 are provided between the cell separation device 232 and, the bioreactor/fermentor 231 to circulate the culture solution 222 using the pump 248. The culture solution circulation channels 220 and 221 are provided with valves 245, 246 and 247 which are opened and closed as necessary at the time of stopping the culture solution circulation, draining the culture solution from the cell separation device 232, etc.

Communication channels 253 and the valve 254 for communicating each gaseous phase are provided between the bioreactor/fermentor 231 and backwash water reservoir 208. The inner pressures of the bioreactor/fermentor 231 and backwash water reservoir 208 can be easily synchronized by opening the valve 254. It is particularly advantageous to adjust the inner pressure of the backwash water reservoir 208 to be slightly lower than that of the bioreactor/fermentor 231 at the initiation of the filtration step.

The medium vessel 233 is a tank for storing a feed medium to be supplied to the bioreactor/fermentor 231 and provided with a mixer 255, a mixer drive motor 256, a pressure gauge 257 for detecting an inner pressure, a pressure control valve 258, and an air supply valve 259. The medium vessel 233 is connected to the backwash water reservoir 208 by way of the valves 242, 260 and communication channels, enabling the delivery of a feed medium to the backwash water reservoir 208. The medium vessel 233 is maintained at a constant pressure by the pressure control valve 258, based on the measured results of the pressure gauge 257. The pressure is typically applied to 0.01 to 0.05 MPa to prevent bacteria, etc., from entering from outside thereinto. Further, the medium vessel 233 is cooled to 5 to 10°C to prevent the stored medium from being deteriorated.

The tank 234 in the analyzer is a tank for storing the filtrate wherein a target material to be produced separated in the cell separation device 232 is dissolved, and provided with a mixer 261, a mixer drive motor 262, a pressure gauge 263 for detecting an inner pressure, a pressure control valves 264 and an air supply valve 265. The tank 234 in the analyzer is connected to the backwash water reservoir 208 by way of the valves 241 and 244 and the communication channel, enabling the reception of the filtrate from the backwash water reservoir 208. The tank 234 in the analyzer is maintained at a constant pressure by the pressure control valve 264, based on the measured results by the pressure gauge 263. The pressure is typically applied to 0.01 to 0.05 MPa to prevent bacteria, etc., from entering the tank from outside. Further, the tank 234 is cooled to 5 to 10°C to prevent the target substance from being deteriorated while stored.

The continuous culture using the system for cultivating cells of the present invention is carried out by repeating the four steps of the animal cell separation technique described earlier, i.e., (1) filtration step, (2) filtrate discharge step, (3) medium delivery step, and (4) backwash step. Thus, a plurality of selectable media each having a different composition and stored in a plurality of medium vessels 233 can be selected based on a state of a cell and supplied to the bioreactor/fermentor 231, whereby the intended product with high quality can be produced with a high yield.

## Claims

1. A system for cultivating cells comprising:
a bioreactor/fermentor (1) for cultivating cells to be cultivated;
a measuring device (4, 5) for measuring the culture cells being cultivated in the bioreactor/fermentor (1) or a component contained in the culture solution; and
a control device (6, 7) for selecting feed medium components to be added to the bioreactor/fermentor (1) from two or more feed media having different composition ratios based on a state of the culture cells determined by a measured value obtained by the measuring device (4, 5),
**characterised in that**
said measuring device (4, 5) is adapted to measure metabolic flux distribution using intracellular metabolic flux analysis, and
said control device (6, 7) is adapted to select the feed medium components at a ratio of each nutrition metabolic rate determined by the metabolic flux distribution in an unsteady state obtained by the measuring device (4, 5).

2. A method for cultivating cells comprising the steps of:
cultivating cells in a culture solution;
measuring the culture cells being cultivated or a component contained in the culture solution; and
selecting feed medium components from two or more feed media having different composition ratios based on a state of the culture cells determined by a measured value obtained in the measuring step and adding the feed medium to the culture solution,
**characterised by**
measuring metabolic flux distribution using intracellular metabolic flux analysis, and
selecting the feed medium components at a ratio of each nutrition metabolic rate determined by the measured metabolic flux distribution in an unsteady state.

3. The system of claim 1, wherein the intracellular metabolic flux analysis is an analysis method using Elementary Metabolite Unit (EMU) or wherein the two or more feed media have different composition ratios of a carbon source and a nitrogen source.

4. The method of claim 2, wherein the two or more feed media have different composition ratios of a carbon source and a nitrogen source or wherein the intracellular metabolic flux analysis is an analysis method using Elementary Metabolite Unit (EMU).

## Patentansprüche

1. System zum Kultivieren von Zellen, umfassend:
einen Bioreaktor / Fermenter (1) zum Kultivieren von zu kultivierenden Zellen;
eine Messvorrichtung (4, 5) zum Messen der Kulturzellen, die in dem Bioreaktor / Fermenter (1) kultiviert werden, oder eines Bestandteils, der in der Kulturlösung enthalten ist; und
eine Steuerungsvorrichtung (6, 7) zum Auswählen eines dem Bioreaktor / Fermenter zuzuführenden Zufuhrmediums aus zwei oder mehr Zufuhrmedien mit unterschiedlichen Zusammensetzungsverhältnissen abhängig von einem Zustand der Kulturzellen,
welcher durch einen von der Messvorrichtung (4, 5) gewonnenen Messwert bestimmt ist,
**dadurch gekennzeichnet, dass**
die Messvorrichtung (4, 5) dazu ausgelegt ist, mittels einer intrazellulären metabolischen Flussanalyse eine metabolische Flussverteilung zu messen, und
die Steuervorrichtung (6, 7) dazu ausgelegt ist, die Bestandteile der Zufuhrmedien in einem Verhältnis der jeweiligen metabolischen Rate zu bestimmen, die durch die metabolische Flussverteilung in einem von der Messvorrichtung (4, 5) ermittelten unstetigen Zustand bestimmt ist.

2. Verfahren zum Kultivieren von Zellen, umfassend die Schritte:
Kultivieren von Zellen in einer Kulturlösung;
Messen der kultivierten Zellen oder eines in der Kulturlösung enthaltenen Bestandteils; und
Auswählen eines Zufuhrmediums aus zwei oder mehr Zufuhrmedien mit unterschiedlichen Zusammensetzungsverhältnissen abhängig von einem Zustand der Kulturzellen, der durch einen im Messschritt erhaltenen Messwert ermittelt wird, und Zugabe des Zufuhrmediums zu der Kulturlösung,
**gekennzeichnet durch**
das Messen einer metabolischen Flussverteilung mittels einer intrazellulären metabolischen Flussanalyse, und
da Auswählen der Bestandteile der Zufuhrmedien in einem Verhältnis der jeweiligen metabolischen Rate, die durch die metabolische Flussverteilung in einem unstetigen Zustand bestimmt ist.

3. System nach Anspruch 1, wobei die intrazelluläre metabolische Flussanalyse ein Analyseverfahren ist, welches die Elementare Metabolit-Einheit (EMU) nutzt oder wobei die zwei oder mehr Zufuhrmedien unterschiedliche Zusammensetzungsverhältnisse einer Kohlenstoffquelle und einer Stickstoffquelle haben.

4. Verfahren nach Anspruch 2, wobei die zwei oder mehr Zufuhrmedien unterschiedliche Zusammensetzungsverhältnisse einer Kohlenstoffquelle und einer Stickstoffquelle haben oder wobei die intrazelluläre metabolische Flussanalyse ein Analyseverfahren ist, welches die Elementare Metabolit-Einheit (EMU) nutzt.

## Revendications

1. Système pour cultiver des cellules comprenant :
un bioréacteur/fermenteur (1) pour cultiver des cellules devant être cultivées ;
un dispositif de mesure (4, 5) pour mesurer les cellules de culture étant cultivées dans le bioréacteur/fermenteur (1) ou un composant contenu dans la solution de culture ; et
un dispositif de commande (6, 7) pour sélectionner des composants de milieu d'alimentation devant être ajoutés au bioréacteur/fermenteur (1) parmi deux milieux d'alimentation ou plus ayant différents rapports de composition sur la base d'un état des cellules de culture déterminé par une valeur mesurée obtenue par le dispositif de mesure (4, 5),
**caractérisé en ce que**
ledit dispositif de mesure (4, 5) est adapté à mesurer une distribution de flux métabolique en utilisant une analyse de flux métabolique intracellulaire, et
ledit dispositif de commande (6, 7) est adapté à sélectionner les composants de milieu d'alimentation à un rapport de chaque taux métabolique de nutrition déterminé par la distribution de flux métabolique dans un état instable obtenue par le dispositif de mesure (4, 5).

2. Procédé pour cultiver des cellules comprenant les étapes de :
culture de cellules dans une solution de culture ;
mesure des cellules de culture étant cultivées ou d'un composant contenu dans la solution de culture ; et
sélection de composants de milieu d'alimentation parmi deux milieux d'alimentation ou plus ayant différents rapports de composition sur la base d'un état des cellules de culture déterminé par une valeur mesurée obtenue à l'étape de mesure et ajout du milieu d'alimentation à la solution de culture,
**caractérisé par**
la mesure d'une distribution de flux métabolique en utilisant une analyse de flux métabolique intracellulaire, et
la sélection des composants de milieu d'alimentation à un rapport de chaque taux métabolique de nutrition déterminé par la distribution de flux métabolique mesurée dans un état instable.

3. Système selon la revendication 1, dans lequel l'analyse de flux métabolique intracellulaire est un procédé d'analyse utilisant une unité de métabolite élémentaire (EMU) ou dans lequel les deux milieux d'alimentation ou plus ont différents rapports de composition d'une source de carbone et d'une source d'azote.

4. Procédé selon la revendication 2, dans lequel les deux milieux d'alimentation ou plus ont différents rapports de composition d'une source de carbone et d'une source d'azote ou dans lequel l'analyse de flux métabolique intracellulaire est un procédé d'analyse utilisant une unité de métabolite élémentaire (EMU).
